# FASCICULE DE BREVET EUROPEEN

(11) **EP 2 626 428 B1**
(45) Date de publication et mention de la délivrance du brevet: **27.05.2015**
(21) Numéro de dépôt: 13154505.5
(22) Date de dépôt: 08.02.2013
(51) Int. Cl.: C12P 7/40, C12P 7/62, C12P 41/00, C07C 62/34, C07C 69/753, C07C 51/08, C07C 67/08, C07C 213/08, C07C 217/58, C07D 223/16

(54) **Procédé de synthèse enzymatique de l'acide (7S)-3,4-dimethoxybicyclo[4.2.0]octa-1,3,5-triene-7-carboxylique ou de ses esters, et application à la synthèse de l'ivabradine et de ses sels**
Enzymatisches Syntheseverfahren von (7S)-3,4-Dimethoxybicyclo[4.2.0]octa-1,3,5-trien-7-Carbonsäure oder ihren Estern, und Anwendung bei der Synthese von Ivabradin und seinen Salzen
Method for enzymatic synthesis of (7S)-3,4-dimethoxybicyclo[4.2.0]octa-1,3,5-triene-7-carboxylic acid or the esters thereof, and use for the synthesis of ivabradine and the salts thereof

(30) Priorité: 09.02.2012 FR 1251195
(43) Date de publication de la demande: 14.08.2013
(73) Titulaire: Les Laboratoires Servier, 92284 Suresnes Cedex (FR)
(72) Inventeur: Pedragosa Moreau, Sandrine, 45100 ORLEANS (FR); Lefoulon, François, 45000 ORLEANS (FR)

(56) Documents cités:
- EP-A1- 2 145 871
- WO-A1-2011/138625
- Paravidino M. et al: "Chapter 8.2.2.1 Resolution of carboxylates with a non-functionalized stereogenic center at the alpha-position" In: Drauz K et al: "Enzyme Catalysis in Organic Synthesis", 1 février 2012 (2012-02-01), Wiley-VCH, Weinheim, Germany, XP002685584, ISBN: 978-3-527-32547-4 vol. 1, pages 266-270, * le document en entier *
- JÖRG PIETRUSZKA ET AL: "Dynamic Enzymatic Kinetic Resolution of Methyl 2,3-Dihydro-1 H -indene-1-carboxylate", EUROPEAN JOURNAL OF ORGANIC CHEMISTRY, vol. 2009, no. 35, 1 décembre 2009 (2009-12-01), pages 6217-6224, XP055041500, ISSN: 1434-193X, DOI: 10.1002/ejoc.200901025
- FAZLENA H ET AL: "Dynamic kinetic resolution: alternative approach in optimizing S-ibuprofen production", BIOPROCESS AND BIOSYSTEMS ENGINEERING, SPRINGER, BERLIN, DE, vol. 28, no. 4, 1 mars 2006 (2006-03-01), pages 227-233, XP019347388, ISSN: 1615-7605, DOI: 10.1007/S00449-005-0024-1
- HAN-YUAN LIN ET AL: "Dynamic kinetic resolution of (R, S)-naproxen 2,2,2-trifluoroethyl ester via lipase-catalyzed hydrolysis in micro-aqueous isooctane", JOURNAL OF MOLECULAR CATALYSIS B: ENZYMATIC, vol. 24-25, 1 octobre 2003 (2003-10-01), pages 111-120, XP055041511, ISSN: 1381-1177, DOI: 10.1016/S1381-1177(03)00145-0

## Description

La présente invention concerne un procédé de synthèse enzymatique du composé de formule (I) : 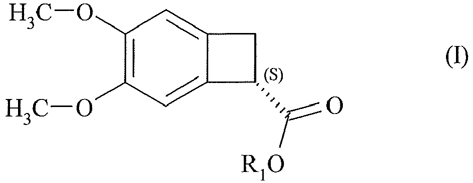 dans laquelle R₁ représente un atome d'hydrogène ou un groupement alkyle C₁-C₆, préférentiellement méthyle,
ainsi que son application à la synthèse de l'ivabradine de formule (II) : 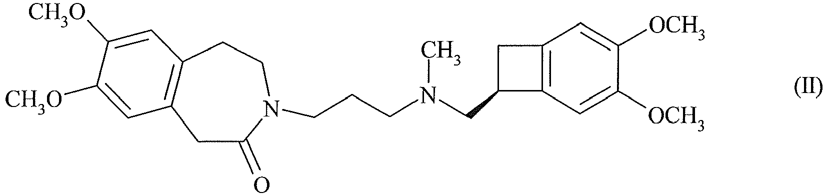 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

Ce brevet décrit la synthèse du chlorhydrate de l'ivabradine à partir du composé de formule (III), la (7S)-1-(3,4-diméthoxy bicyclo[4.2.0] octa-1,3,5-triène 7-yl) N-méthyl méthanamine :

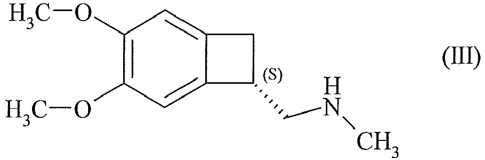

Le composé de formule (III) est un intermédiaire-clé dans la synthèse de l'ivabradine et de ses sels pharmaceutiquement acceptables.

L'art antérieur divulgue plusieurs méthodes d'obtention du composé de formule (III).

Le brevet EP 0 534 859 décrit la synthèse du composé de formule (III) par réduction du nitrile racémique de formule (IV) : 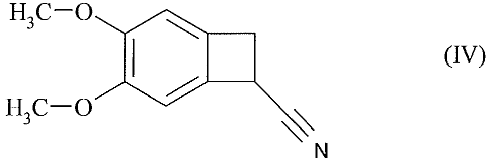 par BH₃ dans le tétrahydrofurane,
suivie de l'addition d'acide chlorhydrique, pour conduire au chlorhydrate de l'amine racémique de formule (V) : 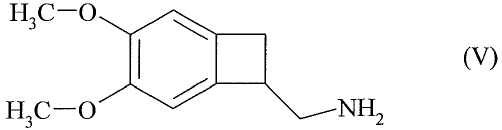 qui est mis en réaction avec du chloroformiate d'éthyle pour conduire au carbamate de formule (VI) : 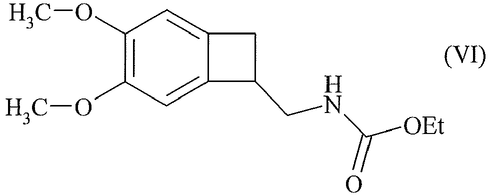 dont la réduction par LiAlH₄ conduit à l'amine méthylée racémique de formule (VII) : 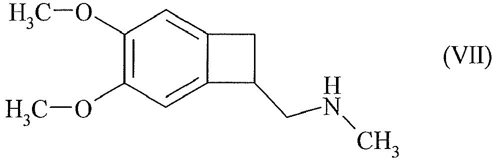 dont le dédoublement à l'aide d'acide camphosulfonique conduit au composé de formule (III). Cette méthode a l'inconvénient de ne conduire au composé de formule (III) qu'avec un rendement très faible de 2 à 3% à partir du nitrile racémique de formule (IV).

Ce rendement très faible est dû au faible rendement (4 à 5%) de l'étape de dédoublement de l'amine secondaire de formule (VII).

Le brevet EP 1 598 333 décrit l'obtention du composé de formule (III) par dédoublement de l'amine primaire racémique de formule (V) en amine optiquement active de formule (VIII) : 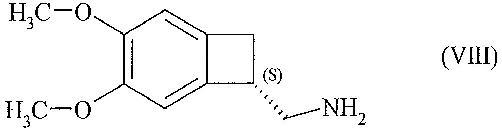 à l'aide d'acide N-acétyl-L-glutamique,
puis méthylation par la même séquence réactionnelle que précédemment (transformation en carbamate, puis réduction).

Le rendement de l'étape de dédoublement est de 39%.

Le brevet EP 2 166 004 décrit l'obtention du composé de formule (III) par résolution optique du nitrile racémique de formule (IV) par chromatographie chirale, pour conduire au nitrile optiquement pur de formule (IX) : 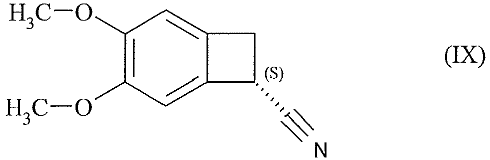 lequel est réduit par NaBH₄ pour conduire à l'amine primaire de formule (VIII), qui est ensuite méthylée par la même séquence réactionnelle que précédemment (transformation en carbamate, puis réduction).

Le rendement de l'étape de dédoublement est de 45%.

Le brevet WO2011138625 décrit l'obtention du composé de formule (III) par hydrolyse du nitrile racémique de formule (IV) pour conduire à l'acide racémique de formule (X) (voir ci-dessous), dont le dédoublement à l'aide d'une base chirale conduit à l'acide optiquement pur de formule (Ia) (voir ci-dessous). L'acide (Ia) est ensuite transformé en le composé de formule (III).

Le problème de la présente invention était d'accéder au composé de formule (III) avec un procédé performant, notamment avec un bon rendement, plus particulièrement sur l'étape de dédoublement.

L'utilisation de la biocatalyse pour permettre l'obtention de molécules chirales apparaît de plus en plus intéressante comme une alternative à la synthèse organique traditionnelle. En effet, l'utilisation d'enzymes présentant des propriétés intrinsèques naturelles telles que la chimio, régio et stéréosélectivité permet d'utiliser les enzymes comme des réactifs d'une chimie verte respectueuse de l'environnement.

Dans le cas décrit ici, l'utilisation d'enzymes hydrolytiques (hydrolases), fonctionnant sans co-facteurs, telles que des lipases (EC 3.1.1.3 dans la classification internationale des enzymes) ou esterases (EC 3.1.1.1) permet l'obtention de composés chiraux -intermédiaires clefs dans la synthèse d'actifs pharmaceutiques - avec des excès énantiomériques élévés et de bons rendements.

Plus spécifiquement, la présente invention concerne un procédé de synthèse du composé de formule (Ia) optiquement pur : 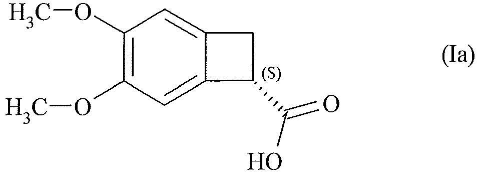 par estérification enzymatique énantiosélective de l'acide racémique ou non optiquement pur de formule (X) : 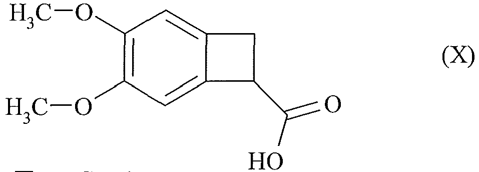 à l'aide d'une lipase de Candida antarctica ou de Pseudomonas fluorescens
dans un mélange d'alcool ROH dans lequel R représente un groupement alkyle C₁-C₆ linéaire ou ramifié, et d'un cosolvant organique,
à une concentration de 5 à 500 g/L, préférentiellement de 100 g à 200 g de composé de formule (X) par litre de mélange de solvants,
à un ratio E/S de 10/1 à 1/100, préférentiellement de 1/5 à 1/10,
à une température de 25°C à 40°C.

Parmi les lipases de *Candida antarctica,* on peut citer à titre d'exemple les lipases immobilisées sur une matrice polymérique, notamment sur résine acrylique telles que Novozym® 435 de la société Novozymes ou SPRIN adsorbed CALB® de la société Sprin Technologies, sur résine polystyrène telles que SPRIN actiplus CALB®, SPRIN acti CALB® ou SPRIN lipo CALB® de la société Sprin Technologies, ou sur résine époxy acrylique telle que SPRIN epobond CALB® de la société Sprin Technologies.

L'alcool ROH est préférentiellement le méthanol ou l'éthanol. Les cosolvants organiques préférés sont l'acétonitrile, le toluène, le MTBE et le n-heptane.

Le ratio cosolvant organique/alcool préféré est de 8/2 à 9/1.

Le schéma de l'estérification enzymatique selon l'invention est le suivant :

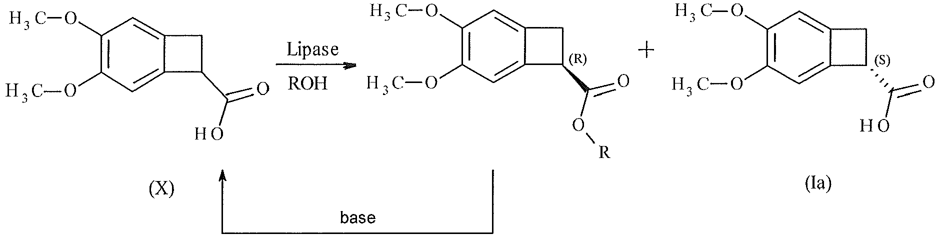

De façon avantageuse, l'ester de configuration (R), produit secondaire de la réaction, peut être saponifié par action d'une base, préférentiellement KOH, DBU, triéthylamine, DABCO, TBD, éthoxyde de sodium, méthoxyde de sodium ou K₂CO₃, en acide racémique de formule (X) pour être recyclé dans le processus d'estérification enzymatique.

Lorsque l'étape de saponification/racémisation est effectuée *in situ,* le procédé selon l'invention est un procédé de dédoublement cinétique dynamique (DKR) qui permet d'obtenir l'acide S de formule (Ia) avec un ee ≥ 98% et un rendement ≥ 65%.

L'acide de formule (Ia) est préférentiellement isolé du milieu réactionnel après un ou plusieurs cycles d'estérification enzymatique.

Un autre aspect de l'invention concerne un procédé de synthèse du composé de formule (Ib) optiquement pur : 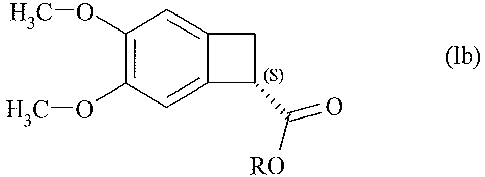 dans lequel R représente un groupement alkyle C₁-C₆ linéaire ou ramifié, préférentiellement méthyle ou éthyle,
par hydrolyse enzymatique énantiosélective de l'ester racémique ou non optiquement pur de formule (XI) : 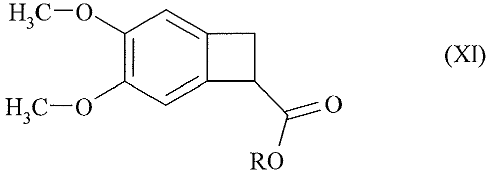 dans lequel R représente un groupement alkyle C₁-C₆ linéaire ou ramifié,
à l'aide d'une lipase de Candida antarctica ou de Pseudomonas fluorescens dans l'eau, dans un tampon à pH=5 à 8 ou dans un mélange de solvant organique et de tampon à pH=5 à 8, à une concentration de 1 à 200 g/L, préférentiellement environ 100 g de composé de formule (XI) par litre de solvant ou mélange de solvants,
à un ratio E/S de 10/1 à 1/100, préférentiellement de 1/5 à 1/10,
à une température de 25°C à 40°C,
suivie de l'isolement de l'ester de formule (Ib).

Parmi les lipases et estérases utilisables dans le procédé d'hydrolyse enzymatique selon la présente invention, on peut citer à titre non limitatif les lipases de *Candida antarctica,* de *Pseudomonas fluorescens,* de *Pseudomonas cepacia,* de *Rhizopus oryzae, d'Aspergillus niger,* de *Mucor javanicus, d'Aspergillus oryzae,* de *Penicillium camemberti,* les estérases de *Rhizopus oryzae,* de *Mucor miehei,* et de *Rhizopus niveus.*

Les lipases préférées selon l'invention sont les lipases de *Candida antarctica* et de *Pseudomonas fluorescens.*

Parmi les lipases de *Candida antarctica,* on peut citer à titre d'exemple les lipases immobilisées sur une matrice polymérique, notamment sur résine acrylique telles que Novozym® 435 de la société Novozymes ou SPRIN adsorbed CALB® de la société Sprin Technologies, sur résine polystyrène telles que SPRIN actiplus CALB®, SPRIN acti CALB® ou SPRIN lipo CALB® de la société Sprin Technologies, ou sur résine époxy acrylique telle que SPRIN epobond CALB® de la société Sprin Technologies.

Lorsque la réaction est effectuée en présence d'un solvant organique, celui-ci est préférentiellement l'acétonitrile, le toluène, le MTBE ou le n-heptane.

Le ratio préféré solvant organique / eau ou tampon va de 8/2 à 9/1.

Le schéma de l'hydrolyse enzymatique selon l'invention est le suivant :

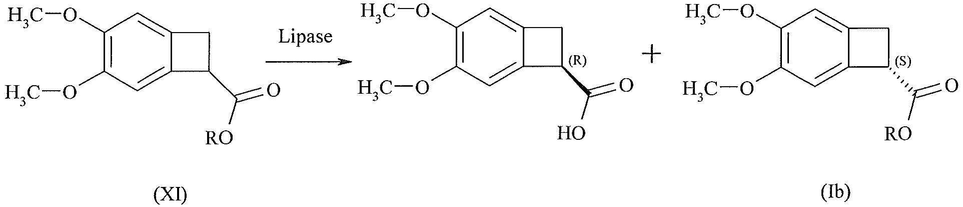

De façon avantageuse, l'acide de configuration (R), produit secondaire de la réaction, peut être racémisé par action d'une base, préférentiellement par action de KOH à chaud, puis l'acide racémique ainsi obtenu peut être alkylé en ester racémique de formule (XI) pour être recyclé dans le processus d'hydrolyse enzymatique.

L'acide de configuration (R), produit secondaire de la réaction, peut alternativement être d'abord alkylé, puis l'ester de configuration (R) ainsi obtenu peut être racémisé par action d'une base, préférentiellement par action de DBU, KOH, triéthylamine, DABCO, TBD, éthoxyde de sodium, méthoxyde de sodium ou K2CO3 pour être recyclé dans le processus d'hydrolyse enzymatique.

Lorsque la racémisation est effectuée à chaud, la température est préférentiellement de 50 à 80°C.

### Définitions

Par composé optiquement pur, on entend composé dont l'excès énantiomérique est supérieur ou égal à 90%.

Par acide ou ester non optiquement pur, on entend acide ou ester dont l'excès énantiomérique est inférieur à 90%.

Par acide ou ester racémique, on entend l'acide ou l'ester sous la forme d'un mélange de deux énantiomères dans un rapport 55:45 à 45:55.

Par estérification énantiosélective d'un acide racémique ou non optiquement pur, on entend estérification préférentielle de l'un des énantiomères du mélange.

Par hydrolyse énantiosélective d'un ester racémique ou non optiquement pur, on entend hydrolyse préférentielle de l'un des énantiomères du mélange.

Un autre aspect de l'invention concerne un procédé de synthèse du composé de formule (III) à partir du nitrile de formule (IV), qui est hydrolysé en acide racémique de formule (X), dont l'estérification enzymatique selon l'invention conduit à l'acide optiquement pur de formule (Ia), qui est ensuite transformé en l'amide optiquement pur de formule (XII) : 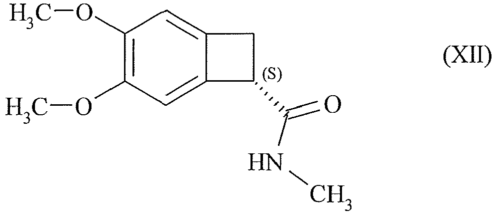 dont la réduction, préférentiellement par BH₃, NaBH₄ ou LiAlH₄, conduit au composé de formule (III).

Un autre aspect de l'invention concerne un procédé de synthèse du composé de formule (III) à partir du nitrile de formule (IV), qui est hydrolyse en acide racémique de formule (X), puis alkylé en ester racémique de formule (XI), dont l'hydrolyse enzymatique selon l'invention conduit à l'ester optiquement pur de formule (Ib), qui est transformé en l'amide optiquement pur de formule (XII) : 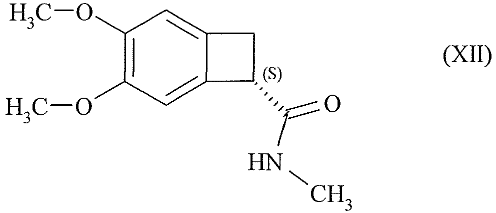 dont la réduction, préférentiellement par BH₃, NaBH₄ ou LiAlH₄, conduit au composé de formule (III).

Le composé de formule (III) est ensuite couplé, soit avec un composé de formule (XIII) : 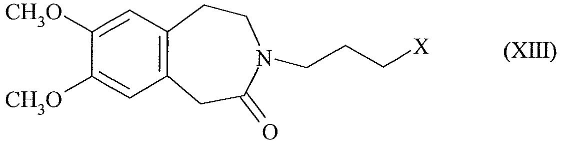 où X représente un atome d'halogène, préférentiellement un atome d'iode,
soit soumis à une réaction d'amination réductrice avec un composé de formule (XIV) en présence d'un agent réducteur : 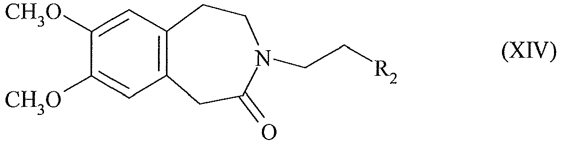 où R₂ représente un groupement choisi parmi CHO et CHR₃R₄,
où R₃ et R₄ représentent chacun un groupement alkoxy (C₁-C₆) linéaire ou ramifié, ou bien forment ensemble avec l'atome de carbone qui les porte un cycle 1,3-dioxane, 1,3-dioxolane ou 1,3-dioxépane,
pour conduire à l'ivabradine, qui est ensuite transformée en un sel d'addition à un acide pharmaceutiquement acceptable, ledit sel étant sous forme anhydre ou hydrate.

Le composé de formule (III) peut être également engagé dans la réaction d'amination réductrice sous la forme de son sel d'addition à un acide pharmaceutiquement acceptable, préférentiellement son chlorhydrate. Dans ce cas, l'ivabradine est obtenue directement sous la forme de chlorhydrate.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique.

Parmi les agents réducteurs utilisables pour la réaction d'amination réductrice entre le composé de formule (III) et le composé de formule (XIV), on peut citer à titre non limitatif les composés donneurs d'hydrure tel que le triacétoxyborohydrure de sodium ou le cyanoborohydrure de sodium, et le dihydrogène en présence d'un catalyseur tel que le palladium, le platine, le nickel, le ruthénium, le rhodium ou un de leurs dérivés, notamment sous forme supportée ou sous forme d'oxydes.

L'agent réducteur préféré pour la réaction d'amination réductrice entre le composé de formule (III) et le composé de formule (XIV) est le dihydrogène catalysé par le palladium sur charbon.

Les exemples ci-dessous illustrent l'invention.

### Abréviations

- ATFA: Acide TriFluoroAcétique
- CCM: Chromatographie sur Couche Mince
- DABCO: 1,4-DiAzaBiCyclo[2.2.2]Octane
- DBU: DiazaBicycloUndécène
- DKR: Dynamic Kinetic Resolution (Dédoublement cinétique dynamique)
- E: Coefficient d'énantiosélectivité
- ee: excès énantiomérique
- éq: équivalent molaire
- HPLC: High Performance Liquid Chromatography (Chromatographie en phase liquide à haute performance)
- MeOH: Méthanol
- MTBE: Methyl Tert-Butyl Ether
- po: pureté optique ou énantiomérique
- ratio E/S: ratio Enzyme/Substrat (g/g)
- RMN: (Spectroscopie) Résonance Magnétique Nucléaire
- SM: Spectrométrie de Masse
- TBD: 1,5,7-TriazaBicyclo-[4.4.0]Dec-5-ène
- THF: TétraHydroFurane
- TMS: TétraMéthylSilane

### EXEMPLE 1 : Acide 3,4-diméthoxybicyclo[4.2.0] octa-1,3,5-triène-7-carboxylique

Mettre en suspension le 3,4-diméthoxybicyclo[4.2.0] octa-1,3,5-triène-7-carbonitrile (11g, 58.1 mmol) dans la soude 1N (70 mL) et chauffer le milieu réactionnel au reflux (110°C) pendant 2h.

Laisser revenir à température ambiante, puis acidifier le milieu par de l'acide chlorhydrique concentré. On observe une précipitation.

Solubiliser le produit dans 200 mL de dichlorométhane puis extraire la phase aqueuse. Sécher sur MgSO₄ et évaporer pour conduire au produit du titre (11.6 g) avec un rendement de 95.9%.

### EXEMPLE 2 : (7S)- Acide 3,4-diméthoxybicyclo[4.2.0] octa-1,3,5-triène-7-carboxylique

0.5 g (c=200g/L) d'acide racémique obtenu à l'Exemple 1 sont solubilisés dans 2.5 mL d'un mélange acétonitrile/ méthanol 8/2.

0.1 g (c=40g/L) de lipase de *Candida antarctica* NOVOZYM 435® (Novozymes Denmark) sont alors ajoutés au milieu (ratio E/S 1/5). Le milieu réactionnel est maintenu à 30°C, sous agitation 220 tr.min rotative pendant 48h.

La réaction est contrôlée par HPLC sur phase chirale dans des conditions permettant de déterminer à la fois les excès énantiomériques de l'ester et de l'acide :
*colonne Chiralpak® IC 250*4.6*
*30% éthanol absolu + 0.1% ATFA + 70% heptane + 0.1% ATFA*
*1ml*/*min 25 °C 288nm*

| | % acide | % ester | **Ee (%) Acide (S)** | **Ee (%) Ester (R)** | **E** |
|---|---|---|---|---|---|
| 18h | 59 | 41 | **66** | **> 99** | **77** |
| 24h | 55 | 45 | **78** | **> 99** | **100** |
| 48h | 51 | 49 | **97** | **> 98** | **890** |

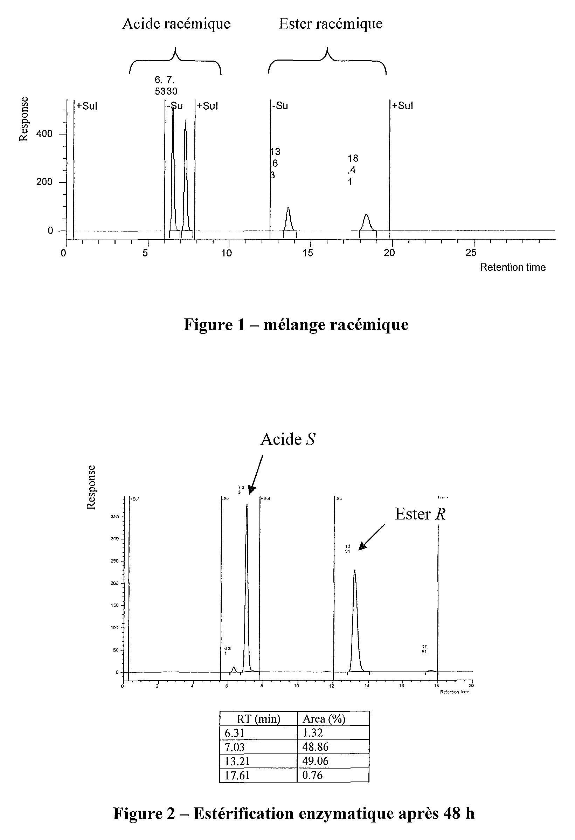

Les chromatogrammes HPLC sur phase chirale des produits racémiques et après 48h sont représentés en Figures 1 et 2.

Après 48h on observe la présence d'ester et d'acide optiquement purs dans un ratio optimal acide/ester proche de 50/50. Le milieu réactionnel est filtré, l'enzyme est lavée par 5 mL de méthanol puis le filtrat est évaporé sous vide. L'acide *S* et l'ester *R* optiquement purs sont séparés par chromatographie sur colonne de silice (éluant : dichlorométhane/méthanol 98/1)

Acide (*S*) : 0.22 g (44%) ; pureté optique > 96% ; [α]²⁰_{D} à 589nm : +57.1° (5mg/ml dans MeOH)

Ester (*R*) : 0.24 g ; pureté optique > 96% ; [α]²⁰_{D} à 589nm : -62.7° (5mg/ml dans MeOH) Rendement global (S + *R*) : 92 %.

### EXEMPLE 3 : 3,4-Diméthoxybicyclo[4.2.0] octa-1,3,5-triène-7-carboxylate de méthyle

Mettre en suspension le (7*R*)-3,4-diméthoxybicyclo[4.2.0] octa-1,3,5-triène-7-carboxylate de méthyle (445 mg) (ee > 96%) dans l'isopropanol (2.5 mL) et ajouter le diazabicycloundécène (58µl - 1.5eq).

Chauffer le milieu réactionnel à 65°C pendant 2h. On observe une racémisation totale au bout de 2h de réaction de l'ester.

Conditions d'analyse :
*colonne Chiralpak® IC 250*4.6*
*30% éthanol absolu + 0.1% ATFA + 70% heptane + 0.1% ATFA*
*1ml*/*min 25°C 288nm*

### EXEMPLE 4 : Acide 3,4-diméthoxybicyclo[4.2.0] octa-1,3,5-triène-7-carboxylique

Mettre en suspension le (7*R*)-3,4-diméthoxybicyclo[4.2.0] octa-1,3,5-triène-7-carboxylate de méthyle (50 mg) (ee > 96%) dans le méthanol (1 mL) et ajouter la potasse (56.1) (25 mg - 2 éq).

Chauffer le milieu réactionnel à 65°C pendant 6h. On observe la saponification de l'ester en acide racémique.

Conditions d'analyse :
*colonne Chiralpak® IC 250*4.6*
*30% éthanol absolu + 0.1% ATFA + 70% heptane + 0.1% ATFA*
*1ml*/*min 25°C 288nm*

### EXEMPLE 5 : (7S)- Acide 3,4-diméthoxybicyclo[4.2.0] octa-1,3,5-triène-7-carboxylique

2 g (c=200 g/L) d'acide 3,4-diméthoxybicyclo[4.2.0] octa-1,3,5-triène-7-carboxylique racémique sont solubilisés dans 20 ml d'un mélange acétonitrile/méthanol (9/1).

0.4 g (c=20 g/L) de lipase de *Candida antarctica* SPRIN actiplus CALB® (Sprin Technologies) sont alors ajoutés au milieu. Le milieu réactionnel est maintenu à 30°C, sous agitation 220 tr.min rotative pendant 24h. L'enzyme est filtrée puis lavée avec du méthanol. 0.5 g de KOH (2eq) sont alors ajoutés au milieu (filtrat) et l'agitation maintenue pendant 6h à 30°C. Le milieu est ensuite évaporé sous vide. Cela permet la racémisation et saponification totale de l'ester R sans racémiser l'acide S. Le résidu est repris par de l'acétate d'éthyle puis lavé avec une solution d'acide citrique 10%. L'extraction à l'acétate d'éthyle n'étant pas suffisante, l'acide soluble dans l'eau est réextrait par une solution de butan-1-ol. Les extraits sont séchés sur MgSO₄ pour donner après évaporation 1.9 g d'acide avec un ratio de 75:25 *(S:R).* Cet acide enrichi énantiomériquement est engagé dans une deuxième réaction enzymatique en présence de 0.2g de lipase. Après 24h à 30°C, l'enzyme est filtrée puis lavée avec du méthanol.

Après évaporation, le résidu est chromatographié sur colonne de Silice (éluant CH₂Cl₂/MeOH de 99/1 à 99/2) pour conduire aux produits suivants :
**Acide (*S*) :** 1.33 g; po > 96 % rendement en acide (75% théorique) : 67%
**Ester (*R*) :** 0.42 g; po > 96 % rendement en ester (25% théorique) : 21 %

Le rendement global de la réaction est de ∼88 %.

### Description RMN et SM de l'acide

¹H RMN (DMSO-d6, ppm / TMS) : 3.17 (dd; 1H; 13.6Hz; 2.4Hz); 3.27 (dd; 1H; 13.6Hz; 5.3Hz); 4.13 (dd; 1H); 3.71 (s ;3H) ; 6.78 (s ; 1H) ; 6.80 (s ; 1H) ; 12.40 (s ; 1H).

SM (EI+) Ion moléculaire M+ à m/z 208.

### Description RMN et SM de l'ester

¹H RMN (DMSO-d6, ppm / TMS) = 3.19 (dd; 1H; 13.6Hz; 2.4 Hz); 3.33 (dd; 1H; 13.6Hz; 5.5Hz); 3.65 (s; 3H); 3.71 (s; 6H); 4.23 (dd; 1H); 6.79 (s; 1H); 6.82 (s;1H).

SM (EI+) Ion moléculaire M+ à m/z 222.

L'enchaînement de réactions est contrôlé par HPLC sur phase chirale dans des conditions permettant de déterminer à la fois les excès énantiomériques de l'ester et de l'acide :
*colonne Chiralpak® IC 250*4.6*
*30% éthanol absolu + 0.1% ATFA + 70% heptane + 0.1% ATFA*
*1ml*/*min 25°C 288nm*

### EXEMPLE 6 : 3,4-Diméthoxybicyclo[4.2.0] octa-1,3,5-triène-7-carboxylate de méthyle

Solubiliser l'acide 3,4-diméthoxybicyclo[4.2.0] octa-1,3,5-triène-7-carboxylique (3g - 14.4 mmol) dans le méthanol et ajouter le chlorure d'acétyle (1.65 g - 21.1 mmol).

Porter le milieu réactionnel à reflux pendant 2h. Une analyse par CCM (éluant dichlorométhane) montre l'absence de l'acide racémique de départ.

Evaporer le milieu réactionnel, reprendre le résidu dans l'acétate d'éthyle et laver la phase organique par NaHCO₃. Evaporer à sec et sécher pour conduire au produit du titre avec un rendement de 97 %.

### EXEMPLE 7 : (7S)-3,4-Diméthoxybicyclo[4.2.0] octa-1,3,5-triène-7-carboxylate de méthyle

2 g (c=100g/L) de 3,4-diméthoxybicyclo[4.2.0] octa-1,3,5-triène-7-carboxylate de méthyle racémique sont solubilisés dans 20 mL d'un mélange acétonitrile/tampon pH=7 80/20.

0.4 g (c=20g/L) de lipase de *Candida antarctica* NOVOZYM 435® (Novozymes Denmark) sont alors ajoutés au milieu (ratio E/S 1/5). Le milieu réactionnel est maintenu à 30°C, sous agitation 230 tr.min rotative.

Après 4h de réaction (pH=5.8), le pH est ajusté à 7.2. Après 24h, l'enzyme est filtrée et lavée par agitation dans le méthanol. Tous les filtrats sont rassemblés, évaporés, et lyophilisés.

Le lyophilisat est repris dans l'acétate d'éthyle, l'agitation est maintenue une nuit puis le milieu réactionnel est filtré et le filtrat est évaporé.

Le résidu est purifié sur colonne de silice (éluant dichlorométhane/méthanol) pour obtenir 0.81 g de l'ester du titre (7*S*) soit avec un rendement de 41%.
[α]²⁰_{D} à 589nm : +64.7° (5mg/ml dans MeOH)

La fraction contenant l'acide est reprise dans l'acétate d'éthyle pour conduire à 0.72g d'acide (7*R*) soit avec un rendement de 36%.
[α]²⁰_{D} à 589nm : -58.8° (5mg/ml dans MeOH)

### EXEMPLE 8 : (7S)-3,4-Diméthoxybicyclo[4.2.0] octa-1,3,5-triène-7-carboxylate de méthyle

Le 3,4-diméthoxybicyclo[4.2.0] octa-1,3,5-triène-7-carboxylate de méthyle racémique (1 mg; c=1 g/L) est solubilisé dans 1 mL de mélange tampon phosphate pH=7/toluène 90/10. 5 mg (c=5g/L) de lipase de *Pseudomonas fluorescens* sont alors ajoutés au milieu (ratio E/S 5/1). Le milieu réactionnel est maintenu à 28°C, sous agitation 220 tr.min rotative pendant 48h.

Le milieu réactionnel est analysé par HPLC en phase inverse et l'énantiosélectivité (ee) de l'ester résiduel est contrôlée par HPLC en phase chirale selon les méthodes décrites ci-dessous
*Conditions d'analyse du milieu réactionnel par HPLC en phase inverse :*
*Kinetex® 2.6µm C18 50*2.140°C 0.6ml*/*min 100% A à 100% B en 5min*
*A (1000 eau+25 ACN+1 ATFA)*
*B (1000 ACN+25 eau+1 ATFA)*
*Conditions d'analyse de l'énantiosélectivité par HPLC en phase chirale :*
*colonne Chiralpak® IC 250*4.6 100% éthanol absolu 1ml*/*min 25°C 288nm*

| *Enantiomère* | *Temps de rétention (min)* |
|---|---|
| *(7R)* | *7.19* |
| *(7S)* | *9.03* |

L'analyse du milieu réactionnel montre une bonne activité hydrolytique (pourcentage d'ester résiduel : 25%).

L'analyse de l'énantiosélectivité montre un ee de 90% pour l'ester (7S).

### EXEMPLE 9 : Acide 3,4-diméthoxybicyclo[4.2.0] octa-1,3,5-triène-7-carboxylique

Mettre en suspension l'acide (7*R*)-3,4-diméthoxybicyclo[4.2.0] octa-1,3,5-triène-7-carboxylique (50 mg - ee> 95%) dans le méthanol (1 mL) et ajouter de l'hydroxyde de potassium (20 mg).

Chauffer le milieu réactionnel à 65°C pendant 24h. On observe la racémisation totale de l'acide.

Conditions d'analyse :
*colonne Chiralpak® IC 250*4.6*
*30% éthanol absolu + 0,1% ATFA + 70% heptane + 0.1% ATFA*
*1ml*/*min 25°C 288nm*

### EXEMPLE 10 : (7S)-3,4-Diméthoxy-N-méthylbicyclo[4.2.0] octa-1,3,5-triène-7-carboxamide

Mettre en suspension le (7S)- acide 3,4-diméthoxybicyclo[4.2.0] octa-1,3,5-triène-7-carboxylique obtenu à l'exemple 5 (300 mg) dans le THF (3 ml) à température ambiante puis ajouter la triéthylamine (200 µl). Le chloroformiate d'éthyle (150 µl) est ajouté lentement au milieu. Le milieu réactionnel précipite (milieu I).

Dans un autre flacon, la méthylamine en solution 2M dans le THF (2.25 ml) est mise sous agitation avec l'eau (1 ml) et la triéthylamine (300 µl). L'agitation est maintenue pendant 20 min puis ce milieu est ensuite additionné au milieu 1 et laissé sous agitation à température ambiante pendant une nuit.

Le mélange réactionnel est ensuite évaporé et purifié par HPLC préparative.

Le (7*S*)-3,4-diméthoxy-N-méthylbicyclo[4.2.0] octa-1,3,5-triène-7-carboxamide est obtenu avec un rendement de 60%.

¹H RMN (DMSO-d6, ppm / TMS) = 2.61 (m; 3H); 3.16 (m; 2H); 3.71 (s; 6H); 4.05 (m; 1H); 6.78 (s; 1H); 6.81 (s;1H); 7.78 (s;1H).

### EXEMPLE 11 : (7S)-3,4-Diméthoxy-N-méthylbicyclo[4.2.0] octa-1,3,5-triène-7-carboxamide

Mettre en suspension le (7S)-3,4-diméthoxybicyclo[4.2.0] octa-1,3,5-triène-7-carboxylate de méthyle (500 mg) dans l'eau puis ajouter lentement, à température ambiante, 20 mL d'une solution de méthylamine à 33% dans l'éthanol absolu.

Après 3h d'agitation, le milieu réactionnel est évaporé. Le résidu obtenu est purifié par HPLC préparative (éluant : eau/acétonitrile/acide trifluoroacétique de 98/2/0.2 à 20/80/0.2) en 30 minutes pour conduire au produit du titre avec un rendement de 70%.

### EXEMPLE 12 : (7S)-3,4-Diméthoxybicyclo[4.2.0]octa-1,3,5-trién-7-yl]-N-méthyl-méthanamine

Mettre en suspension le (7S)-3,4-diméthoxy-N-méthylbicyclo[4.2.0] octa-1,3,5-triène-7-carboxamide (450 mg) dans le tétrahydrofurane (20 mL) puis ajouter lentement 1.6 mL d'une solution de LiAlH₄ 2M dans le tétrahydrofurane au milieu réactionnel à température ambiante. On observe un fort dégagement gazeux et le milieu réactionnel devient limpide. Chauffer le milieu réactionnel à reflux pendant 30 min.

Hydrolyser après le retour à température ambiante, puis extraire par l'acétate d'éthyle. Sécher sur MgSO₄ puis évaporer. Le résidu obtenu est purifié par HPLC préparative (éluant : eau/acétonitrile/acide trifluoroacétique de 98/2/0.2 à 20/80/0.2) en 30 minutes pour conduire au produit du titre avec un rendement de 46%.

¹H RMN (DMSO-d6, ppm / TMS) = 2.60 (m; 3H); 2.85 (m; 1H); 3.15 (m; 1H); 3.25 (dd; 1H); 3.30 (m; 1H); 3.62 (m; 1H); 3.70 (s; 6H); 6.82 (s; 1H); 6.89 (s;1H); 8.48 (s; 1H).

### EXEMPLE 13 : (7S)-3,4-Diméthoxybicyclo[4.2.0]octa-1,3,5-trién-7-yl]-N-méthyl-méthanamine, chlorhydrate

20 mL d'une solution molaire de BH₃ dans le tétrahydrofurane sont ajoutés à température ambiante au mélange de 2,2 g (10 mmol) du (*7S*)-3,4-diméthoxy-N-méthylbicyclo[4.2.0] octa-1,3,5-triène-7-carboxamide dans 45mL de tétrahydrofurane. Après 1h sous agitation, 10mL de la solution de BH₃ dans le tétrahydrofurane sont ajoutés. Après une nuit d'agitation à température ambiante, 20 mL d'éthanol sont additionnés goutte à goutte et le mélange est agité jusqu'à fin de dégagement gazeux (1h environ). 20 mL d'une solution d'acide chlorhydrique dans l'éthanol sont ensuite additionnés goutte à goutte. Après 4h sous agitation, le précipité obtenu (1.2 g de produit du titre) est filtré. Le filtrat est concentré et 0.65g supplémentaires de produit du titre sont obtenus par concrétisation dans un mélange acétate d'éthyle/éthanol 80/20.

Les deux précipités sont réunis pour conduire à 1.85 g du produit du titre (Rendement 77%).

### EXEMPLE 14 : Chlorhydrate de l'ivabradine

Dans un autoclave, charger 5.5 kg de 3-[2-(1,3-dioxolan-2-yl)éthyl]-7,8-diméthoxy-1,3-dihydro-2*H*-3-benzazépin-2-one, 27.5 1 d'éthanol et 550 g de palladium sur charbon.

Purger à l'azote puis à l'hydrogène, chauffer à 55°C, puis hydrogéner à cette température sous une pression de 5 bars jusqu'à absorption de la quantité théorique d'hydrogène.

Ramener ensuite à température ambiante, puis décompresser l'autoclave.

Ajouter ensuite 4 kg du chlorhydrate de la (7*S*)-3,4-diméthoxybicyclo[4.2.0]octa-1,3,5-trién-7-yl]-N-méthylméthanamine, 11 l d'éthanol 5.5 l d'eau et 1 kg de palladium sur charbon. Purger à l'azote puis à l'hydrogène, chauffer à 85°C, puis hydrogéner à cette température sous une pression de 30 bars jusqu'à absorption de la quantité théorique d'hydrogène.

Revenir ensuite à température ambiante, purger l'autoclave, puis filtrer le mélange réactionnel, distiller les solvants puis isoler le chlorhydrate d'ivabradine par cristallisation dans un mélange toluène/1-méthyl-2-pyrrolidinone.

Le chlorhydrate d'ivabradine est ainsi obtenu avec un rendement de 85 % et une pureté chimique supérieure à 99 %.

### EXEMPLE comparatif : Screening de lipases et estérases pour l'hydrolyse enzymatique du 3,4-diméthoxybicyclo[4.2.0] octa-1,3,5-triène-7-carboxylate de méthyle

Le 3,4-diméthoxybicyclo[4.2.0] octa-1,3,5-triène-7-carboxylate de méthyle racémique (1 mg; c=1 g/L) est solubilisé dans 1 mL de mélange tampon phosphate pH=7/toluène 90/10.

5 mg (c=5g/L) de la lipase ou de l'estérase étudiée sont alors ajoutés au milieu (ratio E/S 5/1). Le milieu réactionnel est maintenu à 28°C, sous agitation 220 tr.min rotative pendant 48h.

Le milieu réactionnel est analysé par HPLC en phase inverse et l'énantiosélectivité (*ee*) de l'ester résiduel est contrôlée par HPLC en phase chirale selon les méthodes décrites ci-dessous
*Conditions d'analyse du milieu réactionnel par HPLC en phase inverse :*
*Kinetex® 2.6µm C18 50*2.140°C 0.6ml*/*min 100% à 100% B en 5min*
*A (1000 eau+25 ACN+1 ATFA)*
*B (1000ACN+25 eau+1 ATFA)*
*Conditions d'analyse de l'énantiosélectivité par HPLC en phase chirale :*
*colonne Chiralpak® IC 250*4.6 100% éhanol absolu 1ml*/*min 25°C 288nm*

| *Enantiomère* | *Temps de prétention (min)* |
|---|---|
| *(7R)* | *7.19* |
| *(7S)* | *9.03* |

Les résultats sont résumés dans le tableau suivant :

| Lipase | % ester | % acide | **Ee^{a} (%) ester** | **E^{b}** |
|---|---|---|---|---|
| Lipase Pancréatique de Porc Type II | - | 100 | 0 | - |
| Lipase PS *(Pseudomonas cepacia)* | 55 | 45 | 34 (énantio S) | 3 |
| Lipase AY *30 (Candida rugosa)* | - | 100 | 0 | - |
| Lipase FAP-15 *(Rhizopus oryzae)* | 45 | 55 | 52 (énantio S) | 4 |
| Lipase A6 *(Aspergillus niger)* | 76 | 24 | 68 (énantio R) | 6 |
| Lipase AH *(Pseudomonas cepacia)* | 90 | 10 | 14 (énantio S) | 8 |
| Lipase M « Amano »10 *(Mucor javanicus)* | 60 | 40 | 36 (énantio S) | 5 |
| Lipase *d'Aspergillus oryzae* | 78 | 22 | 64 (énantio S) | 5 |
| Lipase G « Amano » *(Penicillium camemberti)* | 40 | 60 | 26 (énantio R) | 2 |
| Lipase AYS « Amano » *(Candida rugosa)* | 60 | 40 | 4 (énantio R) | 1 |
| Lipase R « Amano » *(Penicillium roqueforti)* | - | 100 | 0 | |
| Esterase de foie de porc | - | 100 | 0 | |
| Esterase de *Rhizopus oryzae* | 40 | 60 | 50 (énantio S) | 3 |
| Esterase de *Mucor miehei* | 79 | 21 | 45 (énantio S) | 6 |
| Esterase de foie de cheval | - | 100 | 0 | |
| Newlase F (*Rhizopus niveus)* | - | 100 | 0 | - |
| **Lipase de *Pseudomonas fluorescens*** | **25** | **75** | **90 (énantio S)** | **6** |
| **Lipase B de *Candida antarctica (Novozym ® 435)*** | **30** | **70** | **94 (énantio S)** | **9** |

| | | | | |
|---|---|---|---|---|
| ^{a} Excès énantiomérique ee (en %) = % énantioE2 - % énantioE1 / % énantio E2 + % énantio E1 (énantio E2 étant l'énantiomère majoritaire) ^{b} coefficient d'énantiosélectivité E = ln[(1-c)(1-ee(S)] / ln[(1-c)(1+ee(S)] ; c= taux de conversion = ee(ester) /ee(ester) + ee(acide) | | | | |

## Revendications

1. Procédé de synthèse du composé de formule (Ia) optiquement pur : 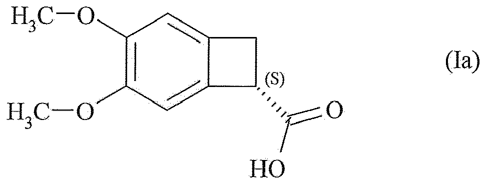 par estérification enzymatique énantiosélective de l'acide racémique ou non optiquement pur de formule (X) : 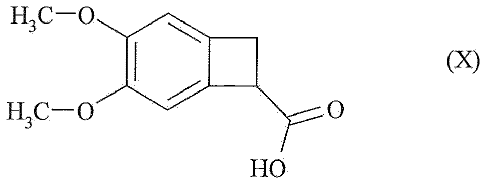 à l'aide d'une lipase de *Candida antarctica* ou de *Pseudomonas fluorescens,*
dans un mélange d'alcool ROH dans lequel R représente un groupement alkyle C₁-C₆ linéaire ou ramifié, et d'un cosolvant organique,
à une concentration de 5 à 500 g/L de composé de formule (X) par litre de mélange de solvants,
à un ratio E/S de 10/1 à 1/100,
à une température de 25°C à 40°C.

2. Procédé de synthèse selon la revendication 1, dans lequel le ratio E/S est de 1/5 à 1/10.

3. Procédé de synthèse selon l'une quelconque des revendications 1 ou 2, dans lequel l'alcool ROH est le méthanol et le cosolvant est l'acétonitrile.

4. Procédé de synthèse selon la revendication 3, dans lequel le ratio acétonitrile/méthanol est de 8/2 à 9/1.

5. Procédé de synthèse selon l'une quelconque des revendications 1 à 4, dans lequel l'ester de configuration (R), produit secondaire de la réaction : 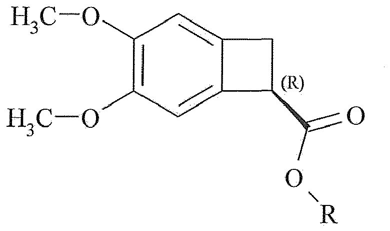 est saponifié par action d'une base en acide racémique de formule (X) pour être recyclé dans le processus d'estérification enzymatique.

6. Procédé de synthèse selon la revendication 5, dans lequel la base est KOH.

7. Procédé de synthèse selon l'une quelconque des revendications 5 ou 6, dans lequel l'étape de saponification/racémisation est effectuée *in situ*.

8. Procédé de synthèse selon l'une quelconque des revendications 1 à 7, dans lequel l'acide de formule (Ia) est isolé après un ou plusieurs cycles d'estérification enzymatique.

9. Procédé de synthèse du composé de formule (Ib) optiquement pur : 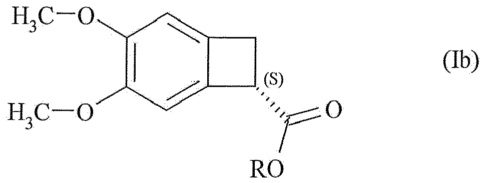 dans lequel R représente un groupement alkyle C₁-C₆ linéaire ou ramifié,
par hydrolyse enzymatique énantiosélective de l'ester racémique ou non optiquement pur de formule (XI) : 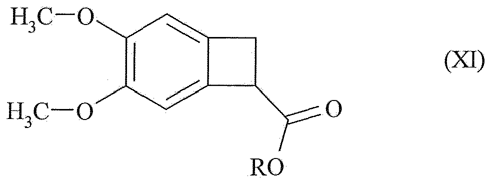 dans lequel R représente un groupement alkyle C₁-C₆ linéaire ou ramifié,
à l'aide d'une lipase de *Candida antarctica* ou de *Pseudomonas fluorescens,* dans l'eau, dans une solution de tampon à pH=5 à 8 ou dans un mélange de solvant organique et
d'eau ou de tampon à pH=5 à 8, à une concentration de 1 à 200 g/L de composé de formule (XI) par litre de solvant ou mélange de solvants,
à un ratio E/S de 10/1 à 1/100,
à une température de 25°C à 40°C,
suivie de l'isolement de l'ester de formule (Ib).

10. Procédé de synthèse selon la revendication 9, dans lequel le ratio E/S est de 1/5 à 1/10.

11. Procédé de synthèse selon l'une quelconque des revendications 9 ou 10, dans lequel R est un groupement méthyle.

12. Procédé de synthèse selon l'une quelconque des revendications 9 à 11, dans lequel la réaction est effectuée dans un mélange d'acétonitrile et de tampon à pH=7.

13. Procédé de synthèse selon la revendication 12, dans lequel le ratio acétonitrile / tampon pH=7 est de 8/2 à 9/1.

14. Procédé de synthèse selon l'une quelconque des revendications 9 à 13, dans lequel l'acide de configuration (R) : 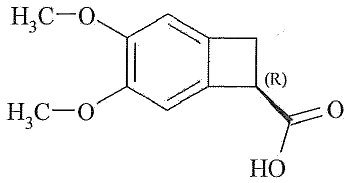 produit secondaire de la réaction,
est racémisé par action d'une base, puis l'acide racémique ainsi obtenu est alkylé en ester racémique de formule (XI) pour être recyclé dans le processus d'hydrolyse enzymatique.

15. Procédé de synthèse selon la revendication 14, dans lequel l'acide de configuration (R) est racémisé par action de KOH à chaud.

16. Procédé de synthèse selon l'une quelconque des revendications 9 à 13, dans lequel l'acide de configuration (R), produit secondaire de la réaction : 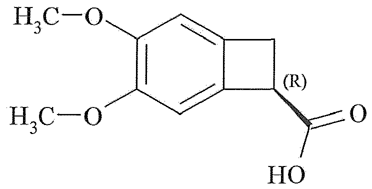 est d'abord alkylé, puis l'ester de configuration (R) ainsi obtenu est racémisé par action d'une base pour être recyclé dans le processus d'hydrolyse enzymatique.

17. Procédé de synthèse selon la revendication 16, dans lequel l'ester de configuration (R) est racémisé par action du DBU à chaud ou de KOH à température ambiante.

18. Procédé de synthèse du composé de formule (III) : 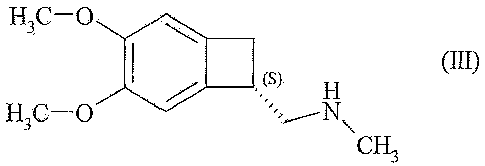 à partir du nitrile de formule (IV) : 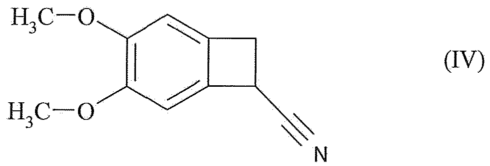 qui est hydrolysé en acide racémique de formule (X) : 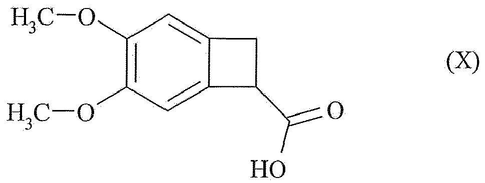 dont l'estérification enzymatique selon l'une quelconque des revendications 1 à 8 conduit à l'acide optiquement pur de formule (Ia) : 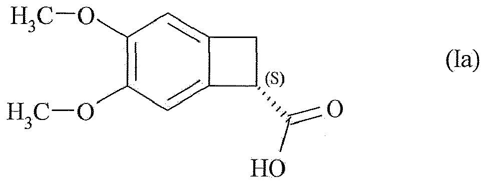 qui est ensuite transformé en l'amide optiquement pur de formule (XII) : 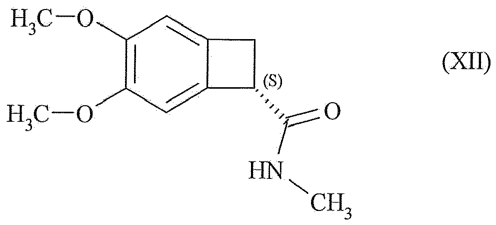 dont la réduction conduit au composé de formule (III).

19. Procédé de synthèse du composé de formule (III) : 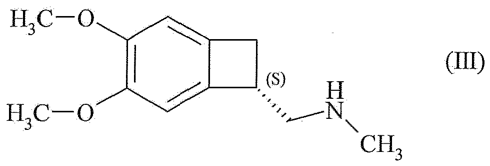 à partir du nitrile de formule (IV) : 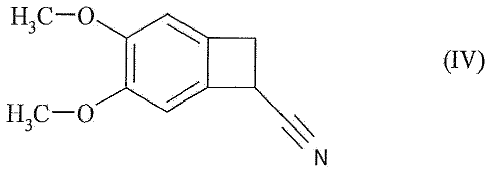 qui est hydrolysé en acide racémique de formule (X) : 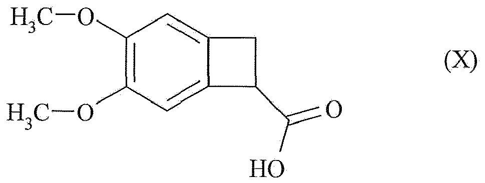 puis alkylé en ester racémique de formule (XI) : 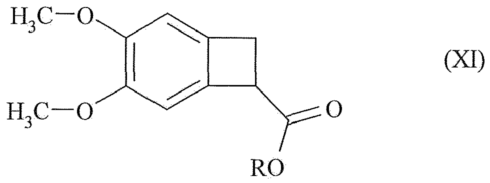 dans lequel R représente un groupement alkyle C₁-C₆ linéaire ou ramifié,
dont l'hydrolyse enzymatique selon l'une quelconque des revendications 9 à 17 conduit à l'ester optiquement pur de formule (Ib) : 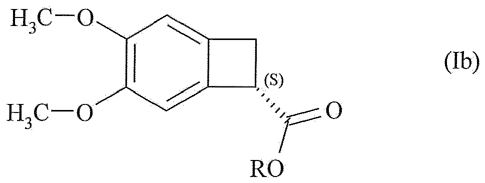 dans lequel R représente un groupement alkyle C₁-C₆ linéaire ou ramifié, qui est transformé en l'amide optiquement pur de formule (XII) : 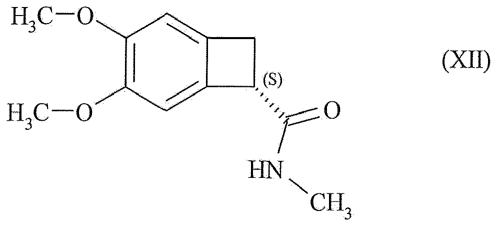 dont la réduction conduit au composé de formule (III).

20. Procédé de synthèse selon l'une quelconque des revendications 18 ou 19, dans lequel la réduction du composé de formule (XII) en composé de formule (III) est effectué par BH₃, NaBH₄ ou LiAlH₄.

21. Procédé de synthèse selon l'une quelconque des revendications 18 à 20, dans lequel le composé de formule (III) est ensuite soit couplé avec un composé de formule (XIII) : 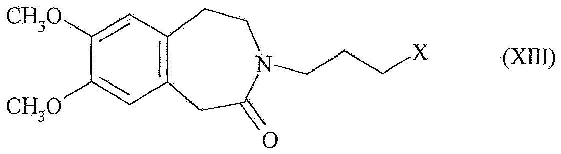 où X représente un atome d'halogène,
soit soumis à une réaction d'amination réductrice avec un composé de formule (XIV) en présence d'un agent réducteur : 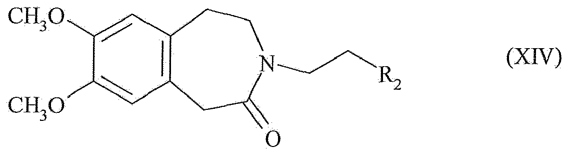 où R₂ représente un groupement choisi parmi CHO et CHR₃R₄,
où R₃ et R₄ représentent chacun un groupement alkoxy (C₁-C₆) linéaire ou ramifié, ou bien forment ensemble avec l'atome de carbone qui les porte un cycle 1,3-dioxane, 1,3-dioxolane ou 1,3-dioxépane,
pour conduire à l'ivabradine, qui est ensuite transformée en un sel d'addition à un acide pharmaceutiquement acceptable, ledit sel étant sous forme anhydre ou hydrate.

22. Procédé de synthèse selon la revendication 21, dans lequel X est un atome d'iode.

23. Procédé de synthèse selon la revendication 21, dans lequel le composé de formule (III) est engagé dans la réaction d'amination réductrice sous la forme de son chlorhydrate, pour conduire à l'ivabradine sous la forme de chlorhydrate.

24. Procédé de synthèse selon l'une quelconque des revendications 21 ou 23, dans lequel la réaction d'amination réductrice avec un composé de formule (XIV) est effectuée en présence de dihydrogène catalysé par le palladium sur charbon.

## Patentansprüche

1. Verfahren zur Synthese der optisch reinen Verbindung der Formel (Ia): 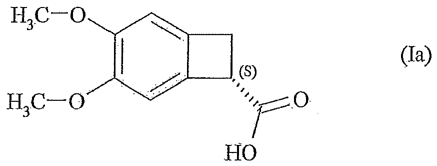 durch enantioselektive enzymatische Veresterung der racemischen oder nicht optisch reinen Säure der Formel (X): 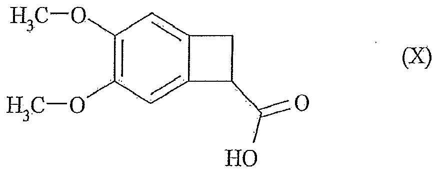 mit Hilfe einer Lipase von *Candida antarctica* oder von *Pseudomonas fluorescens,*
in einer Mischung aus einem Alkohol ROH, worin R eine geradkettige oder verzweigte C₁-C₆-Alkylgruppe bedeutet, und einem organischen Co-Lösungsmittel,
bei einer Konzentration von 5 bis 500 g/l der Verbindung der Formel (X) pro Liter der Mischung der Lösungsmittel,
bei einem E/S-Verhältnis von 10/1 bis 1/100,
bei einer Temperatur von 25 °C bis 40 °C.

2. Syntheseverfahren nach Anspruch 1, worin das E/S-Verhältnis 1/5 bis 1/10 beträgt.

3. Syntheseverfahren nach einem der Ansprüche 1 oder 2, worin der Alkohol ROH Methanol und das Co-Lösungsmittel Acetonitril sind.

4. Syntheseverfahren nach Anspruch 3, worin das Acetonitril/Methanol-Verhältnis 8/2 bis 9/1 beträgt.

5. Syntheseverfahren nach einem der Ansprüche 1 bis 4, worin der als Nebenprodukt der Reaktion gebildete Ester der Konfiguration (R): 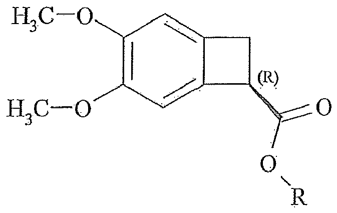 durch Einwirkung einer Base zu der racemischen Säure der Formel (X) verseift wird, die in den Prozess der enzymatischen Veresterung recyclisiert wird.

6. Syntheseverfahren nach Anspruch 5, worin die Base KOH ist.

7. Syntheseverfahren nach einem der Ansprüche 5 oder 6, worin die Stufe der Verseifung/Racemisierung *in situ* durchgeführt wird.

8. Syntheseverfahren nach einem der Ansprüche 1 bis 7 worin die Säure der Formel (Ia) nach einem oder mehreren Zyklen der enzymatischen Veresterung isoliert wird.

9. Verfahren zur Synthese der optisch reinen Verbindung der Formel (Ib): 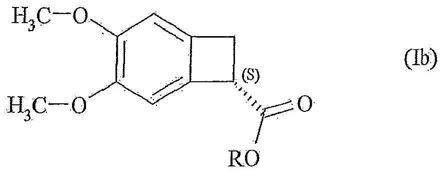 in der R eine geradkettige oder verzweigte C₁-C₆-Alkylgruppe bedeutet,
durch enantioselektive enzymatische Hydrolyse des racemischen oder nicht optisch reinen Esters der Formel (XI): 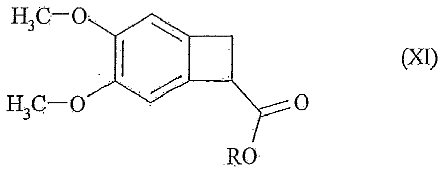 in der R eine geradkettige oder verzweigte C₁-C₆-Alkylgruppe bedeutet,
mit Hilfe einer Lipase von *Candida antarctica* oder von *Pseudomonas fluorescens* in Wasser, in einer Pufferlösung mit einem pH-Wert von 5 bis 8 oder in einer Mischung aus einem organischen Lösungsmittel und Wasser oder einem Puffer mit einem pH-Wert von 5 bis 8 bei einer Konzentration von 1 bis 200 g/l der Verbindung der Formel (XI) pro Liter des Lösungsmittels oder der Lösungsmittelmischung,
bei einem E/S-Verhältnis von 10/1 bis 1/100,
bei einer Temperatur von 25 °C bis 40 °C,
gefolgt von der Isolierung des Esters der Formel (Ib).

10. Syntheseverfahren nach Anspruch 9, worin das E/S-Verhältnis 1/5 bis 1/10 beträgt.

11. Syntheseverfahren nach einem der Ansprüche 9 oder 10, worin R eine Methylgruppe bedeutet.

12. Syntheseverfahren nach einem der Ansprüche 9 bis 11, worin die Reaktion in einer Mischung aus Acetonitril und einem Puffer mit einem pH-Wert von 7 durchgeführt wird.

13. Syntheseverfahren nach Anspruch 12, worin das Verhältnis von Acetonitril zu dem Puffer mit einem pH-Wert von 7 8/2 bis 9/1 beträgt.

14. Syntheseverfahren nach einem der Ansprüche 9 bis 13, worin die Säure der Konfiguration (R): 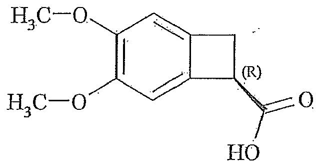 die als Nebenprodukt der Reaktion gebildet wird,
durch Einwirkung einer Base racemisiert wird, wonach die in dieser Weise erhaltene racemische Säure zu dem racemischen Ester der Formel (XI) alkyliert wird, der dann in den Prozess der enzymatischen Hydrolyse recyclisiert wird.

15. Syntheseverfahren nach Anspruch 14, worin die Säure der Konfiguration (R) durch Einwirkung von KOH in der Wärme racemisiert wird.

16. Syntheseverfahren nach einem der Ansprüche 9 bis 13, worin die als Nebenprodukt der Reaktion gebildete Säure der Konfiguration (R): 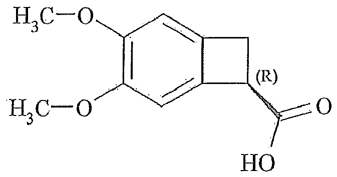 zunächst alkyliert wird, wonach der in dieser Weise erhaltene Ester der Konfiguration (R) durch Einwirkung einer Base racemisiert wird, der dann in den Prozess der enzymatischen Hydrolyse recyclisiert wird.

17. Syntheseverfahren nach Anspruch 16, worin der Ester der Konfiguration (R) durch Einwirkung von DBU in der Wärme oder von KOH bei Raumtemperatur racemisiert wird.

18. Verfahren zur Synthese der Verbindung der Formel (III): 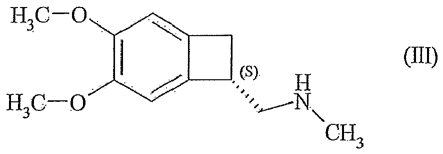 ausgehend von dem Nitril der Formel (IV): 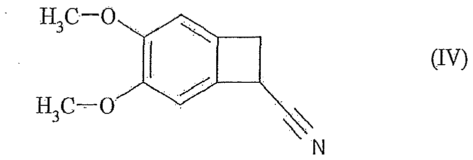 welches zu der racemischen Säure der Formel (X) hydrolysiert wird: 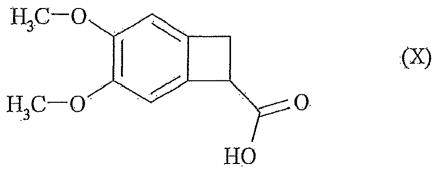 deren enzymatische Veresterung nach einem der Ansprüche 1 bis 8 zu der optisch reinen Säure der Formel (Ia) führt: 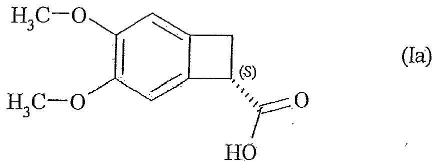 welche anschließend in das optisch reine Amid der Formel (XII) umgewandelt wird: 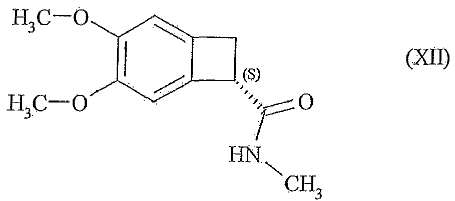 dessen Reduktion zu der Verbindung der Formel (III) führt.

19. Verfahren zur Synthese der Verbindung der Formel (III): 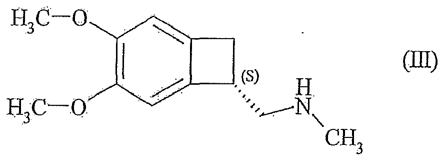 ausgehend von dem Nitril der Formel (IV): 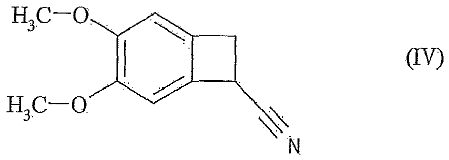 welches zu der racemischen Säure der Formel (X) hydrolysiert wird: 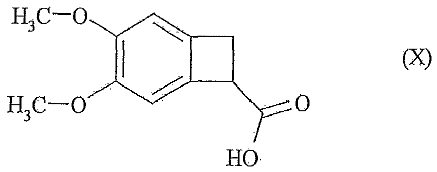 die dann zu dem racemischen Ester der Formel (XI) alkyliert wird: 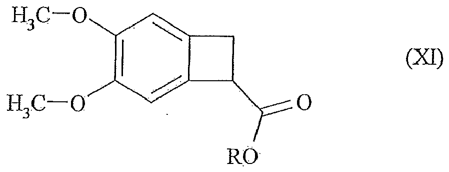 in der R eine geradkettige oder verzweigte C₁-C₆-Alkylgruppe bedeutet,
dessen enzymatische Hydrolyse nach einem der Ansprüche 9 bis 17 zu dem optisch reinen Ester der Formel (Ib) führt: 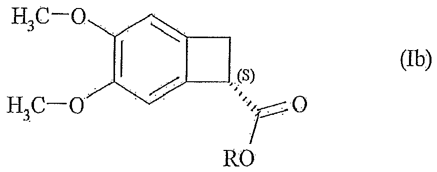 in der R eine geradkettige oder verzweigte C₁-C₆-Alkylgruppe bedeutet, welcher in das optisch reine Amid der Formel (XII) umgewandelt wird: 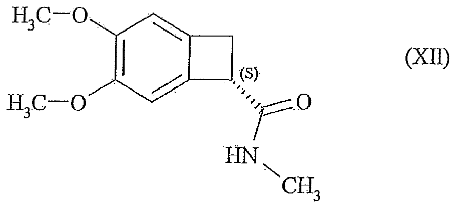 dessen Reduktion zu der Verbindung der Formel (III) führt.

20. Syntheseverfahren nach einem der Ansprüche 18 oder 19, worin die Reduktion der Verbindung der Formel (XII) zu der Verbindung der Formel (III) durch BH₃, NaBH₄ oder LiAlH₄ erfolgt.

21. Syntheseverfahren nach einem der Ansprüche 18 bis 20, worin die Verbindung der Formel (III) anschließend entweder mit einer Verbindung der Formel (XIII): 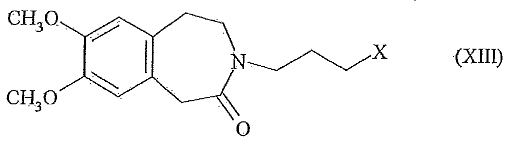 in der X ein Halogenatom bedeutet,
gekuppelt wird oder einer reduzierenden Aminierungsreaktion mit einer Verbindung der Formel (XIV) in Gegenwart eines Reduktionsmittels unterworfen wird: 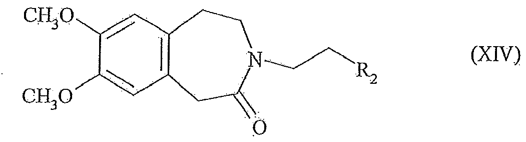 worin R₂ eine Gruppe ausgewählt aus CHO und CHR₃R₄ bedeutet,
worin R₃ und R₄ jeweils eine geradkettige oder verzweigte (C₁-C₆)-Alkoxygruppe bedeuten oder gemeinsam mit dem sie tragenden Kohlenstoffatom einen 1,3-Dioxan-, 1,3-Dioxolan- oder 1,3-Dioxepan-Ring bilden,
zur Bildung von Ivabradin, welches anschließend in eine Additionssalz mit einer pharmazeutisch annehmbaren Säure überführt wird, welches Salz entweder in wasserfreier Form oder in Form des Hydrats vorliegt.

22. Syntheseverfahren nach Anspruch 21, worin X ein Iodatom bedeutet.

23. Syntheseverfahren nach Anspruch 21, worin die Verbindung der Formel (III) in Form ihres Hydrochlorids der reduzierenden Aminierungsreaktion unterworfen wird zur Bildung von Ivabradin in Form des Hydrochlorids.

24. Syntheseverfahren nach einem der Ansprüche 21 oder 23, worin die reduzierende Aminierungsreaktion mit einer Verbindung der Formel (XIV) in Gegenwart von Wasserstoff einer Katalyse mit Palladium-auf-Kohlenstoff bewirkt wird.

## Claims

1. Process for the synthesis of the optically pure compound of formula (Ia): 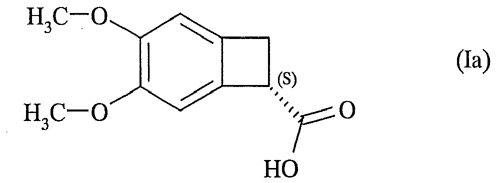 by enantioselective enzymatic esterification of the racemic, or not optically pure, acid of formula (X): 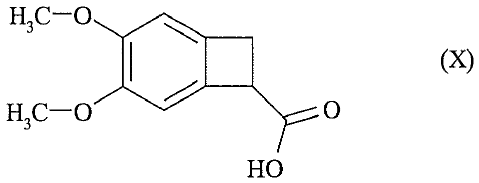 using a lipase of *Candida antarctica* or of *Pseudomonas fluorescens,*
in a mixture of alcohol ROH wherein R represents a linear or branched C₁-C₆alkyl group, and an organic co-solvent,
at a concentration from 5 to 500 g/L of compound of formula (X) per litre of solvent mixture,
at an E/S ratio of from 10/1 to 1/100,
at a temperature from 25°C to 40°C.

2. Synthesis process according claim 1, wherein the E/S ratio is from 1/5 to 1/10.

3. Synthesis process according to either claim 1 or claim 2, wherein the alcohol ROH is methanol and the co-solvent is acetonitrile.

4. Synthesis process according to claim 3, wherein the acetonitrile/methanol ratio is from 8/2 to 9/1.

5. Synthesis process according to any one of claims 1 to 4, wherein the ester of configuration (R), the secondary product of the reaction: 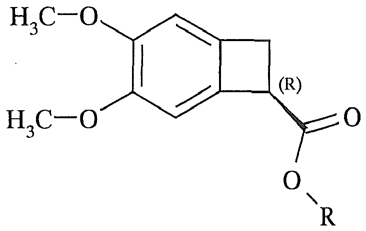 is hydrolysed by the action of a base to form the racemic acid of formula (X) in order to be recycled into the enzymatic esterification process.

6. Synthesis process according to claim 5, wherein the base is KOH.

7. Synthesis process according to either claim 5 or claim 6, wherein the hydrolysis/racemisation step is carried out *in situ.*

8. Synthesis process according to any one of claims 1 to 7, wherein the acid of formula (Ia) is isolated after one or more cycles of enzymatic esterification.

9. Process for the synthesis of the optically pure compound of formula (Ib): 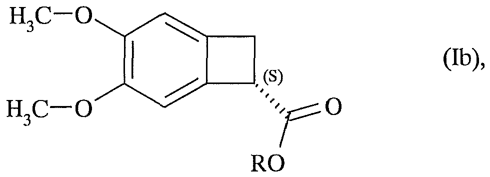 wherein R represents a linear or branched C₁-C₆alkyl group,
by enantioselective enzymatic hydrolysis of the racemic, or not optically pure, ester of formula (XI): 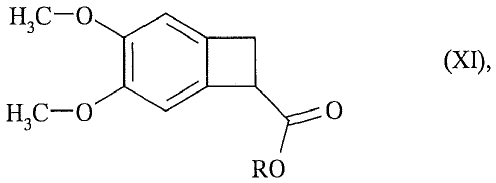 wherein R represents a linear or branched C₁-C₆alkyl group,
using a lipase of *Caradida antarctica* or of *Pseudomonas fluorescens,* in water, in a buffer solution of pH=5 to 8 or in a mixture of organic solvent and water or buffer solution of pH=5 to 8, at a concentration of from 1 to 200 g/L of compound of formula (XI) per litre of solvent or solvent mixture,
at an E/S ratio of from 10/1 to 1/100,
at a temperature from 25°C to 40°C,
followed by isolation of the ester of formula (Ib).

10. Synthesis process according to claim 9, wherein the E/S ratio is from 1/5 to 1/10.

11. Synthesis process according to either claim 9 or claim 10, wherein R is a methyl group.

12. Synthesis process according to any one of claims 9 to 11, wherein the reaction is carried out in a mixture of acetonitrile and buffer of pH=7.

13. Synthesis process according to claim 12, wherein the acetonitrile/buffer pH=7 ratio is from 8/2 to 9/1.

14. Synthesis process according to any one of claims 9 to 13, wherein the acid of configuration (R): 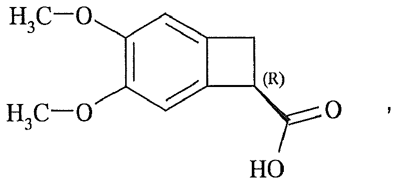 the secondary product of the reaction,
is racemised by the action of a base, and then the racemic acid thereby obtained is alkylated to form the racemic ester of formula (XI) in order to be recycled into the enzymatic hydrolysis process.

15. Synthesis process according to claim 14, wherein the acid of configuration (R) is racemised by the action of KOH in the hot state.

16. Synthesis process according to any one of claims 9 to 13, wherein the acid of configuration (R), the secondary product of the reaction: 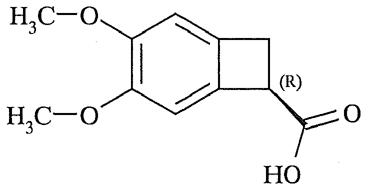 is first alkylated and then the ester of configuration (R) thereby obtained is racemised by the action of a base in order to be recycled into the enzymatic hydrolysis process.

17. Synthesis process according to claim 16, wherein the ester of configuration (R) is racemised by the action of DBU in the hot state or of KOH at ambient temperature.

18. Process for the synthesis of the compound of formula (III): 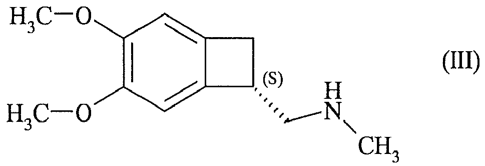 starting from the nitrile of formula (IV): 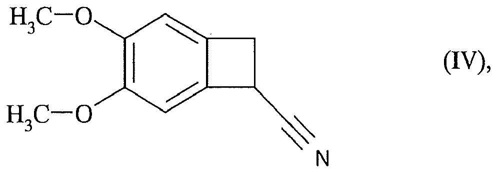 which is hydrolysed to form the racemic acid of formula (X): 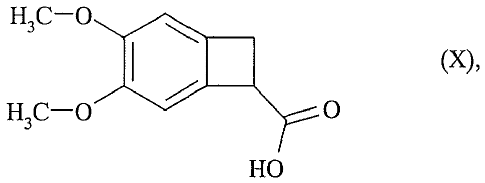 the enzymatic esterification of which in accordance with any one of claims 1 to 8 yields the optically pure acid of formula (Ia): 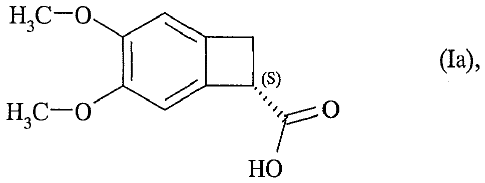 which is then converted into the optically pure amide of formula (XII): 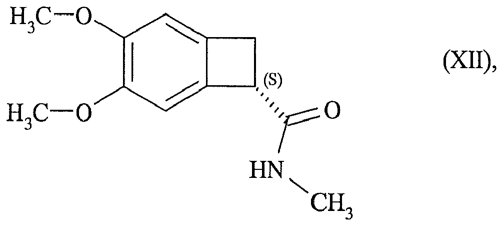 the reduction of which yields the compound of formula (III).

19. Process for the synthesis of the compound of formula (III): 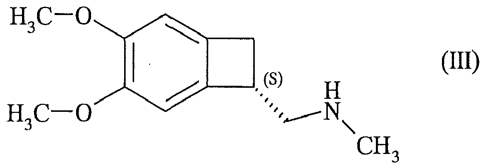 starting from the nitrile of formula (IV): 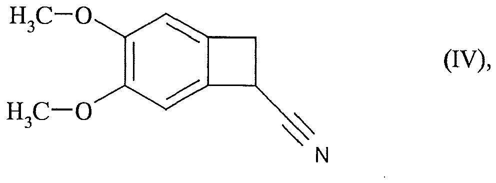 which is hydrolysed to form the racemic acid of formula (X): 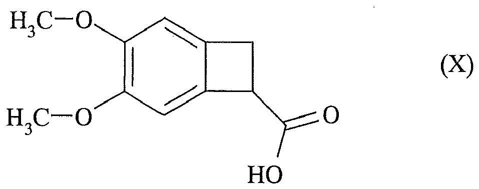 and then alkylated to form the racemic ester of formula (XI): 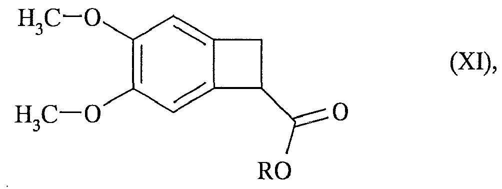 wherein R represents a linear or branched C₁-C₆alkyl group,
the enzymatic hydrolysis of which in accordance with any one of claims 9 to 17 yields the optically pure ester of formula (Ib): 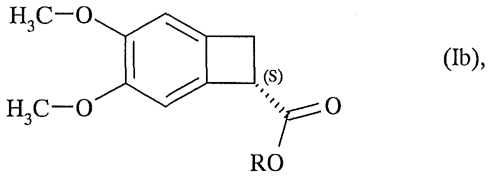 wherein R represents a linear or branched C₁-C₆alkyl group, which is converted into the optically pure amide of formula (XII): 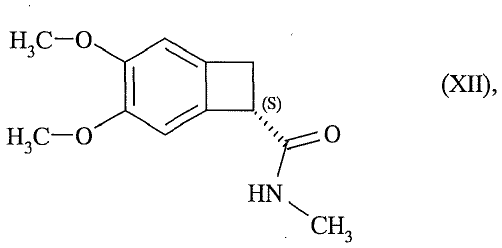 the reduction of which yields the compound of formula (III).

20. Synthesis process according to either claim 18 or claim 19, wherein the reduction of the compound of formula (XII) to form the compound of formula (III) is carried out by BH₃, NaBH₄ or LiAlH₄.

21. Synthesis process according to any one of claims 18 to 20, wherein the compound of formula (III) is subsequently either coupled with a compound of formula (XIII) : 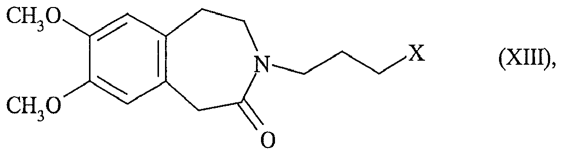 wherein X represents a halogen atom,
or subjected to a reductive amination reaction with a compound of formula (XIV) in the presence of a reducing agent: 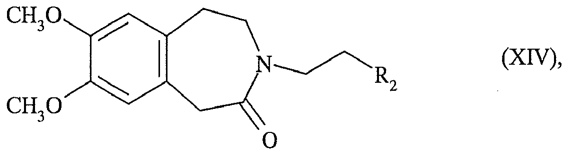 wherein R₂ represents a group selected from CHO and CHR₃R₄,
wherein R₃ and R₄ each represent a linear or branched (C₁-C₆)alkoxy group or form, together with the carbon atom carrying them, a 1,3-dioxane, 1,3-dioxolane or 1,3-dioxepane ring,
to yield ivabradine, which is then converted into an addition salt with a pharmaceutically acceptable acid, said salt being in anhydrous or hydrate form.

22. Synthesis process according to claim 21, wherein X is an iodine atom.

23. Synthesis process according to claim 21, wherein the compound of formula (III) is used in the reductive amination reaction in the form of its hydrochloride to yield ivabradine in the form of the hydrochloride.

24. Synthesis process according to either claim 21 or claim 23, wherein the reductive amination reaction with a compound of formula (XIV) is carried out in the presence of dihydrogen catalysed by palladium-on-carbon.
